# EUROPEAN PATENT APPLICATION

(11) **EP 1 815 973 A1**
(43) Date of publication of application: **08.08.2007**
(21) Application number: 05806773.7
(22) Date of filing: 11.11.2005
(51) Int. Cl.: B32B 7/06, A61J 3/07, B32B 27/00, C12M 3/00, G01N 1/28

(54) **LAMINATE COMPRISING MULTILAYERED FILM BONDED THROUGH HYDROGEN BOND, SELF-SUPPORTING THIN FILM PROVIDED FROM SAID LAMINATE, AND THEIR PRODUCTION PROCESS AND USE**

(30) Priority: 18.11.2004 JP 2004333876
(71) Applicant: Mitsui Chemicals, Inc., Tokyo 105-7117 (JP); Centre National de la Recherche Scientifique, 75016 Paris (FR)
(72) Inventor: ONO, Shoko, Sodegaura-shi, Chiba 2990265 (JP); DECHER, Gero, Strasbourg Cedex F-F67083 (FR)
(74) Representative: HOFFMANN EITLE
(86) International application number: PCT/JP2005/021173
(87) International publication number: WO 2006/054668

(57) **Abstract**

This invention relates to: a laminate comprising a support, a multilayer film assembled via a hydrogen bond thereon, and a thin film thereon; a self-supported thin film obtained from such laminate; a method for producing such laminate comprising allowing a support to come into contact alternately with two or more types of solutions comprising substances to be laminated to form a multilayer film and forming a thin film via a method selected from among vapor deposition, spin coating, and layer-by-layer adsorption; a method for producing a self-supported thin film comprising immersing the laminate in a pH-adjusted aqueous solution to dissolve the multilayer film and to separate the thin film from the support; a thin film for cell culture and a capsule for a controlled-release drug obtained from the laminate; and a sensor, a separation film, a device for catalytic reactions, and a sample slice for transmission assay obtained from such thin film.

## Description

### Technical Field

The present invention relates to a laminate comprising a multilayer film assembled by a hydrogen bond, a self-supported thin film obtained therefrom, and a production method and application of the same. More particularly, the present invention relates to a technique for releasing a thin film comprising substances that are sensitive to acidic or alkaline conditions or temperature changes and that are fragile without losing biological, electronic, magnetic, or optical properties.

### Background Art

In the development of various types of functional devices, such as medical devices, electronic materials, optical materials, or sensors, development of highly regulated thin films is strongly desired for the purpose of miniaturization, multifunctionalization, and integration of such devices. As methods for producing thin films, vacuum deposition, molecular beam epitaxy, solution casting, spin coating, the Langmuir-Blodgett method, layer-by-layer adsorption (US Patent No. 5,208,111; Decher et al., Science, 277, 1232, 1997; Multilayer Thin Films: Sequential Assembly of Nanocomposite Materials; Decher, G. and Schlenoff, J. B., eds., Wiley-VCH: Weinheim, 2003), and other methods are known. In general, thin films obtained by such techniques are often used in combination with supports. If such thin films can be used separate from supports, i.e., as self-supported films, a range of practical applications is expected to further expand. Thus, establishment of a technique for separating a thin film from a support has been awaited.

In general, thin films are obtained via spin coating. Spin coating is a simple method that can be performed at room temperature and ordinary pressure. This technique, however, suffers from drawbacks, such as lack of controllability of a film thickness on the nanometer-level, difficulty in increasing an area, restriction of the shape of a thin film to a planar shape, or frequent damage imposed on a shape at the time of separation.

Meanwhile, several reports have been made on techniques wherein the support surface is coated with an external stimuli-sensitive material, a thin film of interest is provided thereon, and properties of the external stimuli-sensitive material are denatured by the external stimuli to separate the thin film of interest. As an external stimuli-sensitive material that coats the support surface, i) temperature-sensitive poly(N-isopropylacrylamide) (WO 02/08387; EP Patent Publication No. 1264877), ii) acetyl cellulose that is soluble in an organic solvent (US Patent Publication No. 2001/0046564; Kotov et al., Nature Materials, 2, 413, 2003), and the like are used. Document i) discloses that temperature-sensitive poly(N-isopropylacrylamide) is grafted onto the surface of a commercialized culture dish through an electron beam, cells adhere to the surface that becomes hydrophobic at a culture temperature (37°C) but cells spontaneously leave the surface of the culture dish that becomes satisfactorily hydrophilic at a low temperature (32°C) without deteriorating the structures and functions thereof. This breakthrough technique enabled the cell recovery with low damage, which was impossible to perform by an existing cell-recovering technique involving the use of protease such as trypsin. This technique, however, suffers from difficulties in application thereof to substances sensitive to temperature change or in further lamination. Document ii) discloses that a support is coated with acetyl cellulose, alternately laminated films comprising water-soluble polymers and clay minerals are formed thereon, the resulting laminate is immersed in an acetone solution, and acetyl cellulose is solely dissolved. Thus, alternately laminated films can be obtained.

Via layer-by-layer adsorption, an alternately laminated film having a multilayer structure on the support surface can be obtained by preparing an aqueous solution of a positive electrolytic polymer (a cation) and an aqueous solution of a negative electrolytic polymer (an anion) and immersing a support of interest alternately in both solutions. This technique does not require the use of any special apparatus or environmental control, this technique can be performed regardless of the material or shape of the support, and this technique is capable of producing thin layers of large areas. Further, a wide variety of charged substances, such as proteins, clay minerals, semiconductor particles, heteropolyacid, or pigments, can be subjected to the layer-by-layer adsorption technique. If an alternately laminated film that is capable of imparting various functions in accordance with the material to be used could be solely detached from the support, the imparted functions may be used without restrictions imposed by the support. The method ii) described above, however, is not suitable for releasing a thin film comprising a substance that is unstable against an organic solvent as a method for separating a thin film from the support. According to a conventional technique, specifically, a film may be damaged during a process of separating a thin film from a support, and it was difficult to obtain a self-supported thin film comprising substances sensitive to temperature changes or an organic solvent with low damage.

In recent years, a technique that had overcome the above-mentioned drawbacks with the utilization of an alternately laminated film (an alternately laminated film constituted via electrostatic interactions between a polyacrylic acid/polydiallyldimethylammonium copolymer and polystyrene sulfonic acid) that is dissolved at neutral pH (a pH 6 or higher) was disclosed (WO 02/085500; Schlenoff et al., J. Am. Chem. Soc., 123, 5368, 2001). Since biocompatibility of constituents of the dissolved film (an alternately laminated film constituted via electrostatic interactions between a polyacrylic acid/polydiallyldimethylammonium copolymer and polystyrene sulfonic acid) is low, use thereof in the biomaterial field is unfortunately inadequate.

Specifically, development of a method that does not damage the film constituents during the process of separating a thin film from a support and that does not leave unwanted substances after the separation has been awaited for miniaturization, multifunctionalization, and integration of various types of functional devices, such as medical devices, electronic materials, optical materials, or sensors.

In contrast, an alternately laminated film that is constituted via a hydrogen bond has been reported as a material that is dissolved at neutral pH (Sukhishvili et al., J. Am. Chem. Soc., 122, 9550, 2000; Sukhishvili et al., Macromolecules, 35, 301, 2002; Hammond et al., Langmuir, 15, 1360, 1999; Caruso et al., Macromolecules, 36, 2845, 2003). However, there is no report concerning the production of a self-supported film or a production method thereof with the utilization of pH sensitivity of such hydrogen bond.

### Disclosure of the Invention

The present invention provides a self-supported thin film that can be put to a wide range of applications, a laminate comprising a multilayer film assembled via a hydrogen bond, and a production method and applications of the same.

In order to attain such objects, the present inventors had attempted development of a multilayer film that is not dissolved during laminating a thin film but is dissolved upon immersing thereof in water. As a result, they discovered that a multilayer film assembled via a hydrogen bond would be constructed at a low pH level and that such film would be dissolved by merely immersing the same in water at neutral pH (generally pH 4.0 to 9.4, preferably pH 6.0 to 8.0, and more preferably pH 7.2 to 7.5). Further, they also discovered that i) various thin films can be formed on the surface of a pH-responsive multilayer film assembled via a hydrogen bond; ii) upon immersing in water, a multilayer film assembled via a hydrogen bond that is present in a deep portion is selectively dissolved and the thin film is spontaneously separated in water; and iii) removal of the separated thin film from water and drying thereof result in the production of a self-supported film. This has led to the completion of the present invention.

Specifically, the present invention includes the following inventions.
(1) A laminate comprising a support, a multilayer film assembled via a hydrogen bond thereon, and a thin film thereon.
(2) The laminate according to (1), wherein the multilayer film assembled via a hydrogen bond is dissolved in a pH-adjusted aqueous solution and the thin film is separated.
(3) The laminate according to (1) or (2), wherein the multilayer film formed on the support comprises two or more components.
(4) The laminate according to any of (1) to (3), wherein the multilayer film formed on the support comprises a physiologically acceptable polymer.
(5) The laminate according to any of (1) to (4), wherein a thickness of the multilayer film formed on the support is between 0.0005 µm and 10 µm.
(6) The laminate according to any of (1) to (5), wherein the thin film comprises in its structure an alternately-laminated film comprising polyelectrolyte.
(7) The laminate according to any of (1) to (6), wherein the thin film comprises at least one member selected from among proteins, cells, colloidal particles, clay minerals, semiconductor particles, and pigment molecules.
(8) The laminate according to any of (1) to (7), which comprises a barrier layer between the multilayer film and the thin film.
(9) A laminate further comprising a multilayered thin film on the thin film of the laminate according to any of (1) to (8).
(10) A self-supported thin film obtained from the laminate according to any of (1) to (9).
(11) A method for producing the laminate according to any of (1) to (9) comprising allowing a support to come into contact alternately with two or more types of solutions comprising substances to be laminated to form a multilayer film and forming a thin film via a method selected from among vapor deposition, spin coating, and layer-by-layer adsorption.
(12) The production method according to (11), wherein the thin film is formed by allowing the support having the multilayer film to come into contact alternately with two or more types of solutions comprising substances to be laminated.
(13) A method for producing a self-supported thin film comprising immersing the laminate according to any of (1) to (9) in a pH-adjusted aqueous solution to dissolve the multilayer film and to separate the thin film from the support.
(14) A thin film for cell culture obtained from the laminate according to any of (1) to (9).
(15) A capsule for a controlled-release drug obtained from the laminate according to any of (1) to (9).
(16) A separation film obtained from the self-supported thin film according to (10).
(17) A device for catalytic reactions obtained from the self-supported thin film according to (10).
(18) A sample slice for transmission assay obtained from the self-supported thin film according to (10).
(19) A tubular self-supported thin film obtained from the self-supported thin film according to (10).

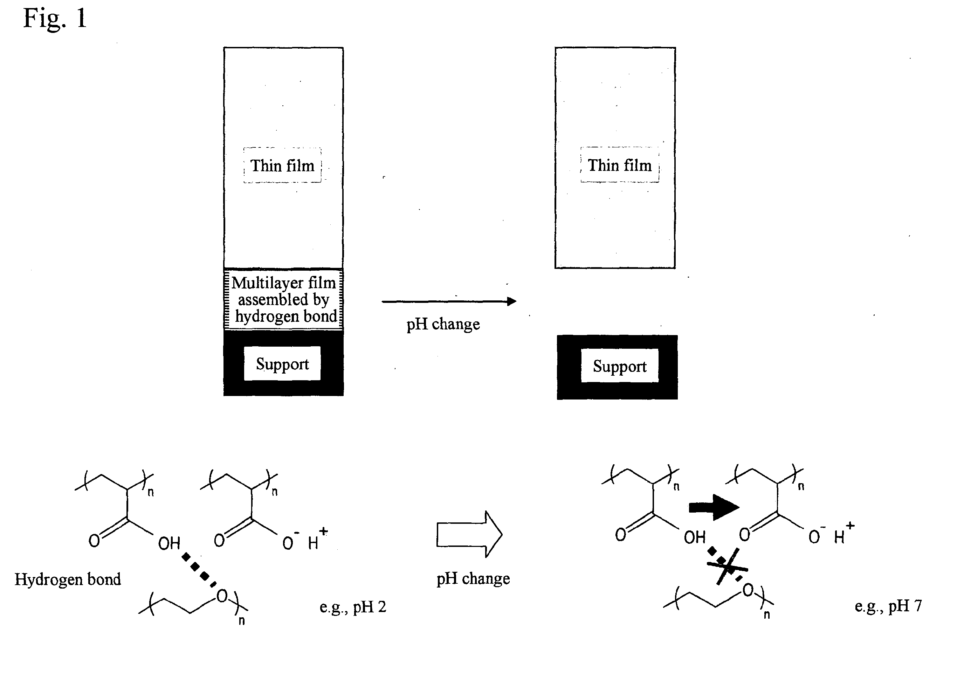

Fig. 1 shows a preferable embodiment of the present invention. According to this embodiment, a self-supported thin film comprising unstable substances is produced with low damage with the utilization of the phenomenon such that a desired thin film , comprising substances sensitive to acidic or alkaline conditions and temperature changes and that are fragile is formed on the surface of a pH-responsive multilayer film assembled via a hydrogen bond, and the resultant is then immersed in water (i.e., pH levels are altered), which results in the selective elution of the multilayer film assembled via a hydrogen bond. Unlike a conventional technique, an elution material is dissolved at neutral pH (e.g., pH 4.0 to 9.4, preferably pH 6.0 to 8.0, and more preferably pH 7.2 to 7.5). Accordingly, a self-supported thin film can be produced without deteriorating biological, electronic, magnetic, or optical properties of unstable substances contained in a desired thin film. If physiologically acceptable polymers, such as polyethylene glycol or polyacrylic acid, i.e., biocompatible and/or biodegradable polymers, are selected as substances constituting materials to be dissolved which cover supports, the resulting film can be suitably used in the biomaterial field.

Hereafter, embodiments of the present invention are described in detail.

Materials of the support that are used in the present invention are not particularly limited. Preferable examples thereof include silicon wafer, quartz, and glass. The shape of the support is not particularly limited, provided that a multilayer film assembled via a hydrogen bond can be directly or indirectly formed on the surface thereof. Specifically, any of the plane, spherical, columnar, or column inner wall surface is applicable. For example, a column inner wall is designated as a support, the inner wall is coated with a multilayer film assembled via a hydrogen bond (a pH-responsive alternately laminated film formed via a hydrogen bond, hereafter it may be referred to as a "hydrogen-bonded film"), alternately laminated films (thin films) constructed by electrostatic interactions are laminated, and the hydrogen-bonded film is dissolved. Thus, a tubular self-supported thin film (an alternately laminated film) is obtained. Also, a particle comprising at least one substance constituting a hydrogen-bonded film is designated as a support, the support is coated with a hydrogen-bonded film, an alternately laminated film formed via electrostatic interactions is laminated, and the hydrogen-bonded film is dissolved. Thus, a capsular self-supported alternately laminated film can be produced. Further, a particle comprising pharmacologically active substances and, laminated on the surface of a hydrogen-bonded film, alternately laminated films constructed via electrostatic interactions enables construction of a system that allows controlled release of pharmacologically active substances in accordance with pH changes (e.g., a controlled-release preparation).

According to the present invention, a substance that constitutes a hydrogen-bonded film generally includes two or more substances, i.e., at least one substance having a bond between a highly electronegative atom such as oxygen or nitrogen and hydrogen, and at least one substance having a highly electronegative atom such as oxygen or nitrogen, capable of forming a hydrogen bond. Such substances can be freely selected by taking applications or use environment into consideration. For example, organic polymers, specifically, polyethylene oxide, polyethylene glycol, polypropylene glycol, polyvinylpyrrolidone, polyvinylpyridine, polyvinyl alcohol, polyacrylic acid, polymethacrylic acid, polylactic acid, polyamino acid (e.g., polyglutamic acid or polylysine), polycaprolactone, or polyurethane can be used. When used in the biomaterial field, physiologically acceptable polymers, i.e., biocompatible and/or biodegradable polymers, polymers that do not adversely affect proteins or cells contained in the pharmacologically active substances or thin films, specifically, polyethylene oxide, polyethylene glycol, polypropylene glycol, polyvinylpyrrolidone, polyacrylic acid, polymethacrylic acid, polylactic acid, or polyamino acid (e.g., polyglutamic acid or polylysine) can be preferably used. Examples of combination of a substance having a bond between a highly electronegative atom such as oxygen or nitrogen and hydrogen, and a substance having a highly electronegative atom such as oxygen or nitrogen, capable of forming a hydrogen bond, include polyacrylic acid and polyethylene glycol, polymethacrylic acid and polyethylene glycol, polyacrylic acid and polyvinylpyrrolidone, and polymethacrylic acid and polyvinylpyrrolidone. The molecular weights of such polymers are generally 1,000 to 5,000,000 g/mol, preferably 10,000 to 1,000,000 g/mol, and more preferably 50,000 to 250,000 g/mol.

A thickness of the hydrogen-bonded film is not particularly limited. When a thin film formed on a laminate is intended to be later extracted as a self-supported film, it is preferable that a distance between a support and a thin film, which is separated by a hydrogen-bonded film, be long enough to such an extent that a bond that would not be dissolved in response to pH changes, e.g., electrostatic interaction, would not take place. Such distance is generally between 0.0005 µm and 10 µm, preferably between 0.005 µm and 1.0 µm, and more preferably between 0.05 µm and 0.5 µm.

In the present invention, a thin film formed on a hydrogen-bonded film is not particularly limited, provided that such film can be formed directly or via a barrier layer on the hydrogen-bonded film, it is stable in water or an aqueous solution at any pH within the range of pH 4.0 to 9.4, and it is useful as a self-supported film. Examples thereof include organic molecules, organic polymers, inorganic molecules, inorganic polymers, organic/inorganic complexes, metals, and metal oxides. Such film may be constituted by one or more regions or may form a multilayer structure comprising a plurality of regions. Part of or the entire film may be a multilayer film. An alternately laminated film is particularly preferable. Any substances can be used for constituting such an alternately laminated film without particular limitation, provided that such substances are two or more oppositely charged substances. For example, polyelectrolyte, proteins, colloidal particles (metal colloids, oxide colloids, or latex colloids), clay minerals, semiconductor particles, heteropolyacids, or pigments can be used. Such substances can be adequately selected in accordance with purposes, applications, desired properties (biological, electronic, magnetic, or optical properties), or use environment. As polyelectrolyte, polymers having charged functional groups on the backbone or side chains can be used, and either polyanions or polycations can be used.

In general, a substance having a negatively chargeable functional group such as sulfonic acid, sulfuric acid, or carboxylic acid is used as a polyanion. For example, conductive polymers such as polystyrene sulfonate (PSS), polyvinyl sulfate (PVS), dextran sulfate, chondroitin sulfate, hyaluronic acid, polyacrylic acid (PAA), polymethacrylic acid (PMA), polymaleic acid, polyfumaric acid, poly(1-(4-(3-carboxy-4-hydroxyphenylazo)benzenesulfonamido)-1,2-ethanediyl) (PAZO), poly(anilinepropanesulfonic acid) (PAPSA), sulfonated polyaniline (SPAN), poly(thiophene-3-acetic acid) (PTAA), poly(2-acrylamido-2-methyl-1-propanesulfonic acid) (PAMPSA), polythiophene, or polyaniline, chromophoric polymers, liquid crystal polymers, or biopolymers such as DNA are used. Preferably, polystyrene sulfonate (PSS), polyacrylic acid (PAA), or the like can be used.

In general, a substance having a positively chargeable functional group such as a quaternary ammonium or amino group can be used as a polycation. For example, polyethyleneimine (PEI), polyallylamine hydrochloride (PAH), polydiallyldimethylammonium chloride (PDDA), polyvinylpyridine (PVP), polylysine, polystylenemethylenediethylmethylamine (PSMDEMA), a precursor of poly(phenylene vinylene) (Pre-PPV), polymethylpyridylvinyl (PMPyV), or protonated poly(p-pyridyl vinylene) (R-PHPyV) can be used. Preferably, polyethyleneimine (PEI), polyallylamine hydrochloride (PAH), or the like can be used.

Examples of polyanion-polycation complexes preferably include a polyallylamine hydrochloride (PAH)-polystyrene sulfonate (PSS) complex and a polyallylamine hydrochloride (PAH)-polyacrylic acid (PAA) complex.

Such organic polymer ions are soluble in water or soluble in a mixture of water and an organic solvent. As an electronic, display material or the like, when conductivity is imparted to a self-supported alternately laminated film, conductive polymers, such as poly(phenylenevinylene) (PPV), can also be used. When the thin film is a polymeric film, the molecular weight of the polymer to be used is generally 1,000 to 5,000,000 g/mol, preferably 10,000 to 1,000,000 g/mol, and more preferably 50,000 to 250,000 g/mol. When a biosensor or an enzyme reaction field is constructed, proteins such as cytochrome c, lysozyme, histone F3, myoglobin, bacteriorhodopsin, albumin or glucoamylase, various types of deoxyribonucleic acids (DNAs) or ribonucleic acids (RNAs), or charged biopolymers such as charged polysaccharides such as pectin can be used. In order to improve mechanical strength of a self-supported alternately laminated film, clay minerals, such as montmorillonite, hectorite, kaoline, or raponite, can be preferably used.

As the thin films described above, thin films formed via vapor deposition (e.g., vacuum deposition), spin coating, or other means can also be used, in addition to the alternately laminated films formed via layer-by-layer adsorption.

The thin film of the self-supported alternately laminated film of the present invention may comprise substances sensitive to acidic or alkaline conditions, temperature changes, or organic solvents and that are fragile, according to need. The term "substances sensitive to acidic or alkaline conditions, temperature changes, or organic solvents and that are fragile" refers to substances, the inherent biological, electronic, magnetic, or optical properties of which are deteriorated due to pH changes, temperature changes, or organic solvents. Examples of such substances include proteins, cells, catalysts, and metals. An example thereof is an enzyme catalyst comprising a protein, the catalytic action thereof is expressed at pH 4.0 to 9.4 (preferably 6.0 to 8.0, and more preferably 7.2 to 7.5) or at 35°C. to 40°C but is not expressed or is deteriorated under other conditions.

The thickness of the thin film is generally between 0.0005 µm and 10 µm, preferably between 0.005 µm and 1.0 µm, and more preferably between 0.05 µm and 0.5 µm.

According to the present invention, even when the thin film comprises the "substances sensitive to acidic or alkaline conditions, temperature changes, or organic solvents and that are fragile," self-supported thin films that satisfactorily retain, or retain preferably 80% or more of the inherent biological, electronic, magnetic, or optical properties of such substances can be obtained with little damage. For example, proteins are accumulated and recovered without being denatured.

According to the present invention, a barrier layer is preferably provided between a hydrogen-bonded film and a thin film. This barrier layer prevents a solution, which contains a substance sensitive to acidic or alkaline conditions or temperature changes and that is fragile (neutral pH, generally at pH 4.0 to 9.4, preferably pH 6.0 to 8.0, and more preferably pH 7.2 to 7.5), from being dispersed in the already-formed thin film and prevents the hydrogen-bonded film from being dissolved at the time of production of a thin film of interest. Substances similar to those constituting the thin film can be used as substances constituting the barrier layer. Branched polymers or laminar compounds, such as branched polyethyleneimine or montmorillonite, that particularly effectively coat the surfaces are preferably used. The thickness of the barrier layer is generally 5 nm or more, preferably 10 nm or more, and more preferably between 50 nm and 10 µm.

The laminate of the present invention comprises a support, a hydrogen-bonded film (that is dissolved in accordance with pH), and a thin film (that is not dissolved in accordance with pH). Preferably, the laminate comprises a support, a hydrogen-bonded film, a barrier layer, and a thin film. The order of laminating the support, the hydrogen-bonded film, (the barrier layer,) and the thin film is limited; however, the hydrogen-bonded film, (the barrier layer,) and the thin film can be formed again on the surface of the resulting laminate. Also, the number of repetition of the lamination of the hydrogen-bonded film, (the barrier layer,) and the thin film is not limited. Such construction is also preferable in the present invention. In addition, a thin film can be further provided on a laminate of the support, the hydrogen-bonded film, (the barrier layer,) and the thin film, as described above. In the present invention, for example, a laminate comprising a support, (hydrogen-bonded film 1, (barrier layer 1,) thin film 1, and (hydrogen-bonded film 2, (barrier layer 2,) and, thin film 2) is constructed so that the pH level at which elution begins differs between hydrogen-bonded film 1 and hydrogen-bonded film 2, and pH levels are gradually altered. Thus, two types of thin films 1 and 2 can be released at different pH levels in a stepwise manner. In the thin film constituted by organic polymers, polymer chains are intertwined with each other. If the hydrogen-bonded film is thin enough, accordingly, two types of alternately laminated films (thin films) formed by electrostatic interactions that sandwich the hydrogen-bonded film are intertwined each other, they interact after the elution of the hydrogen-bonded film so that they are not separated from the under layer. If hydrogen-bonded film 1 and hydrogen-bonded film 2 are thin enough among the support, (hydrogen-bonded film 1, (barrier layer 1,) thin film 1), and (hydrogen-bonded film 2, (barrier layer 2,) and thin film 2), for example, a laminate comprising a support, (barrier layer 1/thin film 1), and (barrier layer 2/thin film 2) is obtained. Such laminate is useful when thin film 1 and thin film 2 are temporality isolated from each other and then brought into contact again. In the case of three-dimensional cell culture techniques as disclosed in WO 02/08387 or EP Patent Publication No. 1264877, for example, a plurality of types of cell sheets are prepared, and the resulting sheets are adequately layered. In the case of the aforementioned technique, however, each of a plurality of types of cells is cultured in a respective thin film while being isolated through hydrogen-bonded films, and the hydrogen-bonded film used for isolation is dissolved to obtain a cell laminate of interest.

In the method for producing the laminate of the present invention, methods for forming a hydrogen-bonded film, a barrier layer, and a thin film on the support are not particularly limited. Preferably, all layers can be formed by bringing two or more types of solutions into contact alternately with the support surface. For example, the dipping method described in Decher et al., Science, 277, 1232, 1997, the spraying method described in Schlenoff et al., Langmuir, 16 (26), 9968, 2000, or spin coating described in Lee et al., Langmuir, 19 (18), 7592, 2003 or J. Polymer Science, part B, polymer physics, 42, 3654, 2004 can be employed. When the alternately laminated films of water-soluble organic polymers are formed via the spray method, for example, the concentration of the aqueous solution is generally 0.01 % to 40%, and preferably 0.1 % to 10%, by weight, the contact time between the laminate surface and the aqueous solution is generally between 1 to 60 seconds, and preferably between 3 to 30 seconds, and the distance between the laminate surface and the spray nozzle is generally between 3 cm and 15 cm, and preferably between 5 cm and 8 cm. After the organic polymer solutions are sprayed, the support is washed with a solution that contains no polymer molecule to wash away polymers that have been excessively adsorbed on the support. Alternately laminated films are formed by repeating the processes of polymer solution spraying and washing. A pH level of a solution may be determined so that the hydrogen-bonded film is not dissolved when forming the hydrogen-bonded film and the barrier layer, and an optimal pH level may be employed for a substance to be contained in the thin film of interest (i.e., the alternately laminated films formed via electrostatic interactions).

According to the method for obtaining a self-supported thin film with low damage with the utilization of the aforementioned laminate, the laminate is immersed in an aqueous solution, the hydrogen-bonded film is then dissolved, and a thin film formed via electrostatic interactions is released in an aqueous solution. The duration between immersing and release of the thin film is generally between 5 seconds and 24 hours, preferably between 5 minutes and 24 hours, and more preferably between 5 minutes and 60 minutes. A pH level of the aqueous solution in which the film is to be immersed is adjusted to a level equivalent to or higher than the pH level at which a hydrogen bond of the hydrogen-bonded film is dissociated. For example, a hydrogen bond in a hydrogen-bonded film comprising polyacrylic acid and polyethylene oxide begins to dissociate at pH 3.6 or higher, and that in a hydrogen-bonded film comprising polymethacrylic acid and polyethylene oxide begins to dissociate at pH 4.6 or higher. If all the substances that constitute the hydrogen-bonded film to be released are biocompatible and biodegradable, such film is effective in terms of physiological safety and thus is preferably used in the field of biomaterials or the like. By altering the number of laminating hydrogen-bonded films or thin films, the speed of dissolving the hydrogen-bonded film, i.e., the speed of releasing the self-supported thin film, can be controlled. Also, adequate selection of polymer types for the hydrogen-bonded film enables controlling of a pH level at which a film is dissolved.

In addition to a pH-adjusted aqueous solution, any solution in which the hydrogen-bonded film can be dissolved can be used without limitation, as long as the hydrogen-bonded film can be dissolved. For example, urea can be used.

The self-supported thin film resulting from the laminate can be obtained as a thin film having an industrially applicable size, i.e., a thin film of several cm size. The film can be prepared to have a thickness of 50 nm to several µm with an error of approximately 10% (in general, an error is within 2% to 3 % or 5% in the case of vapor deposition or layer-by-layer adsorption, and an error is approximately 10% in the case of spin coating).

The laminate of the present invention can be used for producing a thin film for cell culture or a capsule for a controlled-release drug. When the laminate is used for producing a thin film for cell culture, for example, cell culture may then be performed in accordance with a conventional technique on the outermost surface of the hydrogen-bonded film of the laminate, and the entire laminate may be immersed in water. Thus, the thin film that had been subjected to cell culture is spontaneously released. In accordance with types of cells to be cultured, culture conditions including a medium must be optimized. Addition of protease trypsin that is generally added for recovery may not be required. Thus, cells can be obtained as a thin film sheet without losing the structures and functions of such cells. When the laminate is used for producing a capsule for a controlled-released drug, the substance to be released is mixed with either one of the polyacrylic acid or polyethylene glycol solution, a hydrogen-bonded film comprising the substance to be released, polyacrylic acid, and polyethylene glycol (pH 2.00) is formed, a polymer electrolytic laminate comprising polyallylamine hydrochloride and polysulfonate is prepared with the regulation of density on the surface of the hydrogen-bonded film comprising polyacrylic acid as a outermost layer, encapsulated (pH 2.00), and then immersed in an aqueous solution, the pH level of which has been adjusted to 7.00. This causes the hydrogen-bonded film to dissolve and the substance to be released to come out. Thus, it can be used as a controlled-release capsule.

The self-supported thin film according to the present invention can be used for preparing a separation film, a device for catalytic reactions, or a sample slice for transmission assay. In such a case, types of polymers to be used or substances to be introduced may be adequately determined to use the film for preparing a separation film or a device for catalytic reactions. When anionic or cationic polyelectrolyte are selected, for example, charged particles of smoke or negative ions are adsorbed by the Coulomb's force. If a substance that adsorbs specific gas is selected as a component of the laminate, a nonadsorptive substance is selectively transmitted, and the film is thus used as a separation film. When proteins are selected as components of the laminate, proteins can be dispersed and immobilized in the laminate. Thus, the resultant can be used as a reaction vessel for enzyme reactions. Since the obtained self-supported thin film does not require a support, the mixture before separation, the product after separation, or reactants and products are not limited. Since the thin film obtained by layer-by-layer adsorption could not be separated from the support in the past, transmission assay could not be performed. The present invention enabled the performance of transmission electron microscopy or spectroscopy (e.g., ultraviolet and visible or infrared spectroscopy) of the alternately laminated films.

### Brief Description of the Drawings

Fig. 1 shows a preferable embodiment of the present invention.
Fig. 2 shows changes in the thickness of the hydrogen-bonded film and dependence of the growth behavior thereof on the molecular weight of polyacrylic acid, wherein symbols indicate as follows.
   ○: molecular weight of polyacrylic acid of 250,000 g/mol
   □: molecular weight of polyacrylic acid of 100,000 g/mol
   ◇: molecular weight of polyacrylic acid of 30,000 g/mol
   ×: molecular weight of polyacrylic acid of 2,000 g/mol
Fig. 3 shows elution of the hydrogen-bonded film in an aqueous solution in Example 5.
Fig. 4 shows changes in the thickness of the hydrogen-bonded film and dependence of the growth behavior thereof on the molecular weight of polyacrylic acid, wherein symbols indicate as follows.
   •: molecular weight of polyacrylic acid of 250,000 g/mol
   □ : molecular weight of polyacrylic acid of 100,000 g/mol
   ◆ : molecular weight of polyacrylic acid of 30,000 g/mol
Fig. 5 shows elution of the hydrogen-bonded film in an aqueous solution in Examples 12 to 16, wherein symbols indicate as follows.
   ◆: Example 12 (the number of times of contact with an aqueous polyacrylic acid solution and with an aqueous polyethylene glycol solution: 3 times)
   □ : Example 13 (the number of times of contact with an aqueous polyacrylic acid solution and with an aqueous polyethylene glycol solution: 5 times)
   ■: Example 14 (the number of times of contact with an aqueous polyacrylic acid solution and with an aqueous polyethylene glycol solution: 7 times)
   ○: Example 15 (the number of times of contact with an aqueous polyacrylic acid solution and with an aqueous polyethylene glycol solution: 9 times)
   •: Example 16 (the number of times of contact with an aqueous polyacrylic acid solution and with an aqueous polyethylene glycol solution: 11 times)

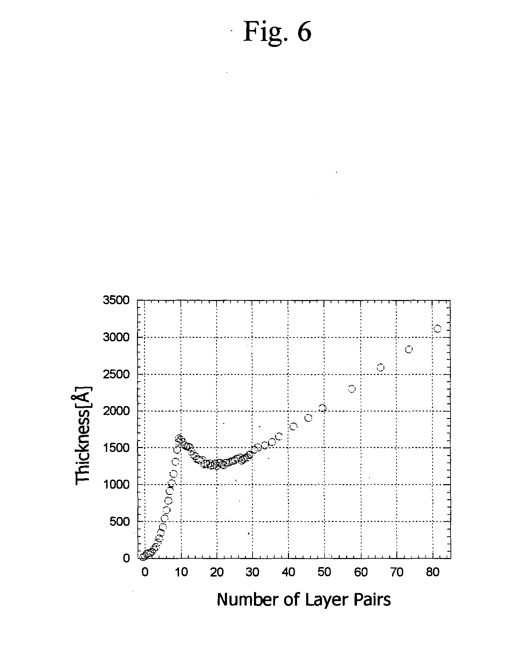

Fig. 6 shows changes in a film thickness when an alternately laminated film is formed via electrostatic interactions on the surface of the hydrogen-bonded film.

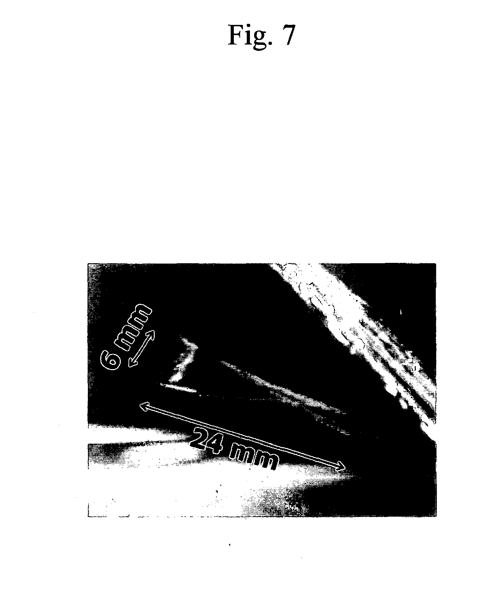

Fig. 7 is a photograph showing a thin film that has been spontaneously released (in water).

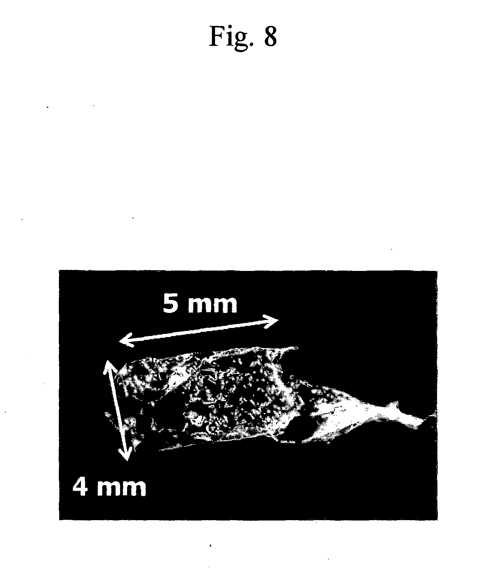

Fig. 8 is a photograph showing a self-supported film that has been spontaneously released and then dried (in the air).

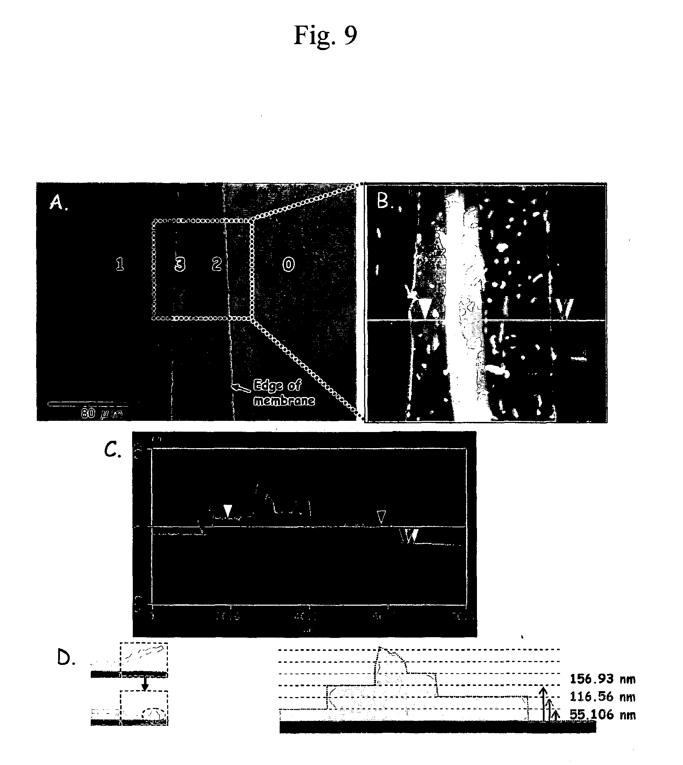

Fig. 9 is a diagram for illustrating a film-thickness measurement of the alternately laminated films formed via electrostatic interactions remaining undissolved using an atomic force microscope.

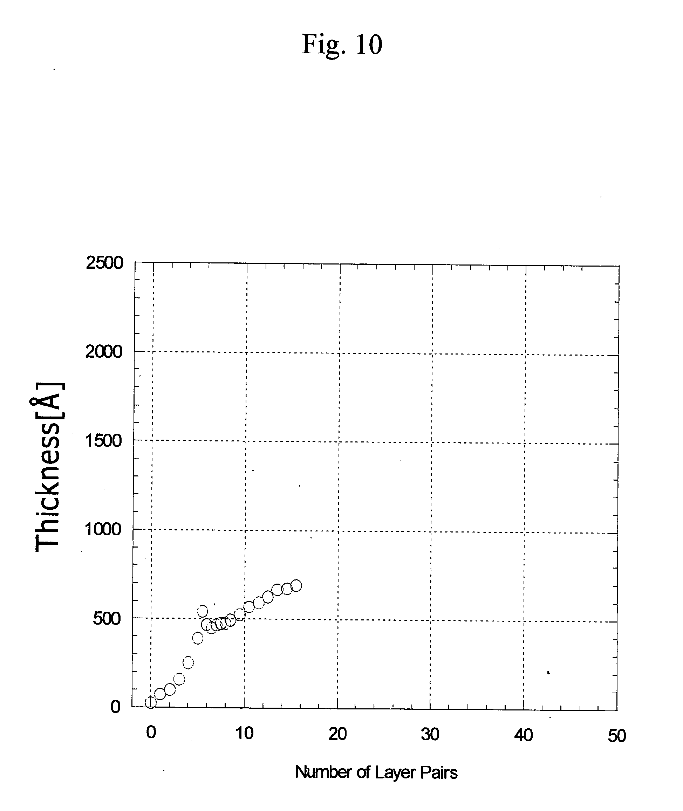

Fig. 10 shows growth of the hydrogen-bonded film/the electrostatically interactive film (when not separated).

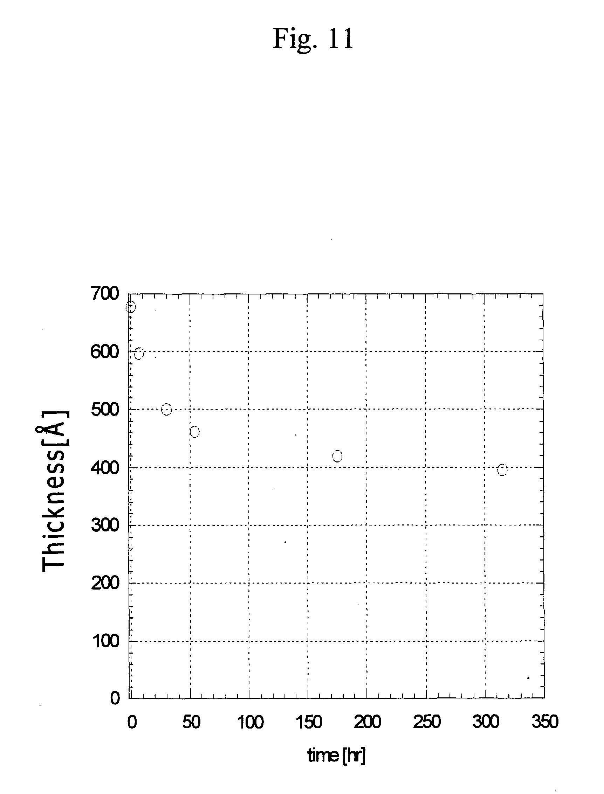

Fig. 11 shows elution of the hydrogen-bonded film in an aqueous solution in Example 21.

This description includes part or all of the contents as disclosed in the description and/or drawings of Japanese Patent Application No. 2004-333876, which is a priority document of the present application.

### Preferred Embodiments of the Invention

Hereafter, the present invention is described in greater detail with reference to the examples. In the following examples, ultrapure water (Milli-Q, Millipore GmbH) was used as "water." pH was adjusted using hydrochloric acid. In the examples, the film thickness was measured via ellipsometry (PLASMOS SD2300) at the wavelength of 632.8 nm (a helium-neon laser) and at the angle of incidence of 45 degrees. After lamination or elution, the resultant that had been blow dried with a nitrogen gas was designated as an analyte, the same spot was subjected to measurement 5 times in the air, and the average thereof was determined to be a film thickness.

### Examples 1 to 4: Production of a hydrogen-bonded film comprising polyacrylic acid and polyethylene glycol

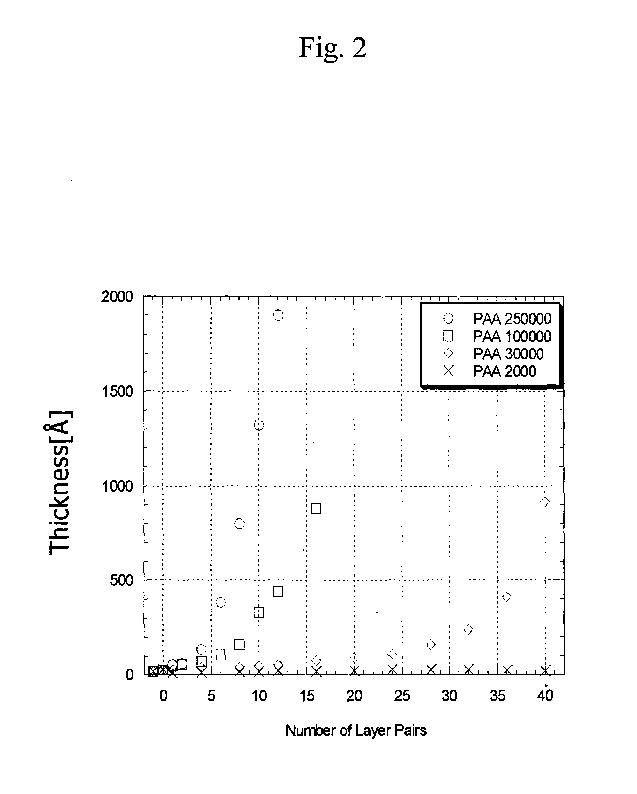

The silicon wafer surface that had been washed with water and with acetone and then blow dried with a nitrogen gas (width: 24 mm; length: 76 mm) was brought into contact alternately with an aqueous solution of water-soluble polyacrylic acid (molecular weight: 2,000 g/mol (Example 1), 30,000 g/mol (Example 2), 100,000 g/mol (Example 3), 250,000 g/mol (Example 4); concentration: 100 mg/100 ml; pH = 2) and with an aqueous solution of water-soluble polyethylene glycol (molecular weight: 18,000 g/mol; concentration: 100 mg/100 ml; pH = 2) by the spray method (contact time with solution: 30 seconds; distance from the spray: 10 cm; sprayed with a polymer solution and then washed with an aqueous solution adjusted to pH 2 for 20 seconds) to prepare a hydrogen-bonded film. Fig. 2 is a chart showing changes in the thickness of the obtained hydrogen-bonded film and dependence of the growth behavior on the molecular weight of polyacrylic acid. In this case, the thickness of each layer is found to exponentially increase with the molecular weight of polyacrylic acid between 30,000 and 250,000 g/mol. The larger the molecular weight, the more the film thickness increases.

### Example 5: Elution of the hydrogen-bonded film

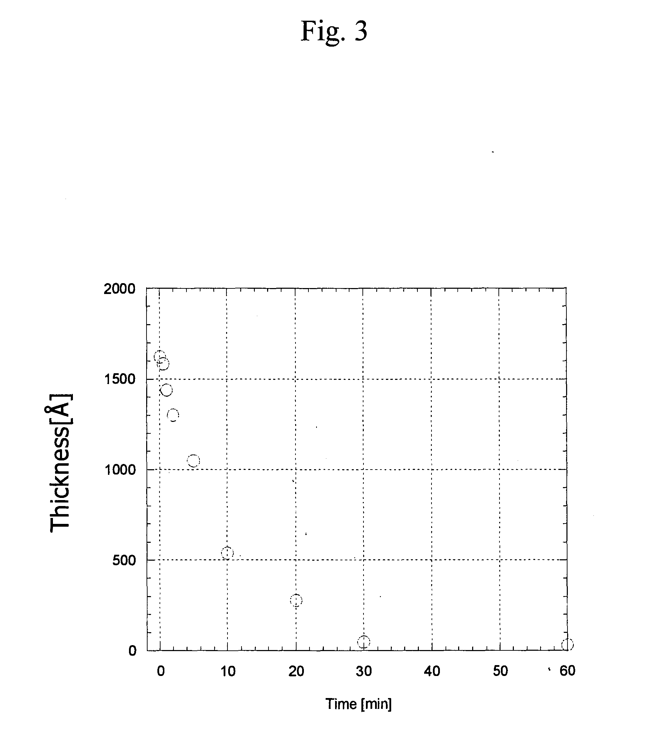

The hydrogen-bonded film obtained by the method of Example 4 was immersed in an aqueous solution (pH 7.4) and then removed therefrom to measure changes in the film thickness. The results are shown in Fig. 3. At pH 7.4, elution is substantially completed within about 30 minutes.

### Examples 6 to 8: Preparation of a hydrogen-bonded film comprising polyacrylic acid and polyethylene glycol

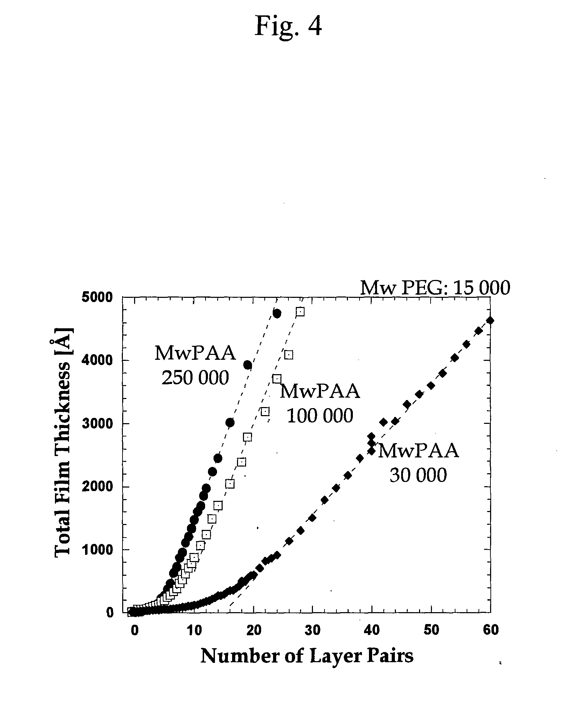

The silicon wafer surface that had been washed with water and with acetone and then blow dried with a nitrogen gas (width: 24 mm; length: 76 mm) was first brought into contact with an aqueous solution of water-soluble polyethyleneimine (molecular weight: 25,000 g/mol; without pH control) by the spray method (contact time with solution: 30 seconds; distance from the spray: 10 cm; sprayed with an aqueous solution of polyethyleneimine and then washed with water at neutral pH for 20 seconds) to prepare a first layer. The surface thereof was brought into contact alternately with an aqueous solution of water-soluble polyacrylic acid (30,000 g/mol (Example 6), 100,000 g/mol (Example 7), 250,000 g/mol (Example 8), concentration: 100 mg/100 ml; pH = 2) and with an aqueous solution of water-soluble polyethylene glycol (molecular weight: 15,000 g/mol; concentration: 100 mg/100 ml; pH = 2) by the spray method (contact time with solution: 30 seconds; distance from the spray: 10 cm; sprayed with a polymer solution and then washed with an aqueous solution adjusted to pH 2 for 20 seconds) to prepare a hydrogen-bonded film. Fig. 4 is a chart showing changes in the thickness of the obtained hydrogen-bonded film and dependence of the growth behavior on the molecular weight of polyacrylic acid. In this case, the thickness of each film is found to increase with the molecular weight of polyacrylic acid between 30,000 and 250,000 g/mol. The larger the molecular weight, the more the film thickness increases.

### Examples 9 to 11: Preparation of a hydrogen-bonded film comprising polyacrylic acid and polyethylene glycol

The silicon wafer surface that had been washed with water and with acetone and then blow dried with a nitrogen gas (width: 24 mm; length: 76 mm) was brought into contact alternately with an aqueous solution of water-soluble polyacrylic acid (30,000 g/mol (Example 9), 100,000 g/mol (Example 10), 250,000 g/mol (Example 11); concentration: 100 mg/100 ml; pH = 2) and with an aqueous solution of water-soluble polyethylene glycol (molecular weight: 15,000 g/mol; concentration: 100 mg/100 ml; pH = 2) by the spray method (contact time with solution: 30 seconds; distance from the spray: 10 cm; sprayed with a polymer solution and then washed with an aqueous solution adjusted to pH 2 for 20 seconds) to form a hydrogen-bonded film. Changes in the thickness of the obtained hydrogen-bonded film and the growth behavior thereof were substantially the same as those shown in Fig. 4.

### Examples 12 to 16: Elution of hydrogen-bonded film

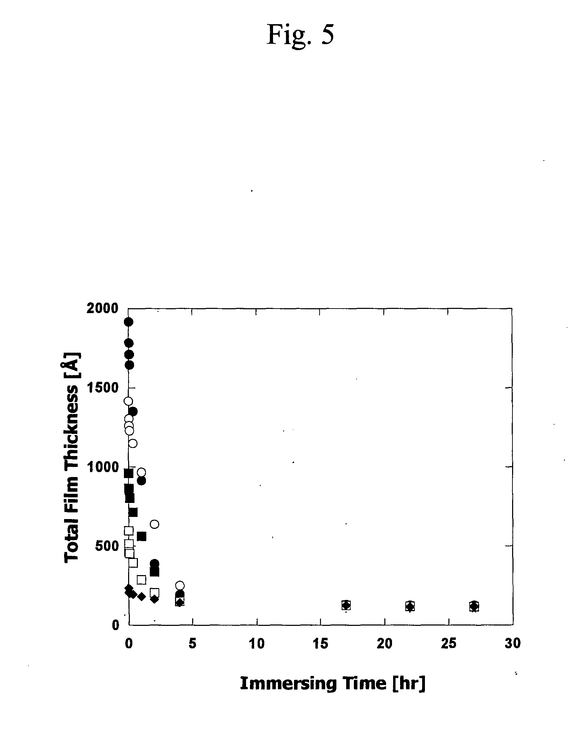

The silicon wafer surface that had been washed with water and with acetone and then blow dried with a nitrogen gas (width: 24 mm; length: 76 mm) was first brought into contact with an aqueous solution of water-soluble polyethyleneimine (molecular weight: 25,000 g/mol, without pH control) by the spray method (contact time with solution: 30 seconds; distance from the spray: 10 cm; sprayed with an aqueous polyethyleneimine solution and then washed with water at neutral pH for 20 seconds) to prepare a first layer. The surface thereof was brought into contact alternately with an aqueous solution of water-soluble polyacrylic acid (250,000 g/mol; concentration: 100 mg/100 ml; pH = 2) and with an aqueous solution of water-soluble polyethylene glycol (molecular weight: 15,000 g/mol; concentration: 100 mg/100 ml; pH = 2) by the spray method (contact time with solution: 30 seconds; distance from the spray: 10 cm; sprayed with a polymer solution and then washed with an aqueous solution adjusted to pH 2 for 20 seconds) 3 times (Example 12), 5 times (Example 13), 7 times (Example 14), 9 times (Example 15), and 11 times (Example 16) to form hydrogen-bonded films. The hydrogen-bonded films obtained by the methods of Examples 12 to 16 were immersed in an aqueous solution (pH about 7.0) and then removed therefrom to measure changes in film thickness. The results are shown in Fig. 5. At pH 7.0, elution was substantially completed within approximately 30 minutes to several hours.

### Example 17: Construction of alternately laminated films on surfaces of hydrogen-bonded films via electrostatic interactions

On the surface of the hydrogen-bonded film obtained by the method of Example 8, an alternately laminated film was formed via electrostatic interactions. Fig. 6 shows changes in the film thickness. In such a case, the formation of the hydrogen-bonded film was completed with polyacrylic acid, the film surface exposed a negative charge, and lamination of alternately laminated films formed via electrostatic interactions was initiated from a polycation, i.e., polyallylamine. The alternately laminated films formed via electrostatic interactions were constructed by bringing the films into contact alternately with an aqueous solution of water-soluble polyallylamine hydrochloride (molecular weight: 70,000 g/mol; concentration: 27.4 mg/100 ml; pH = 2) and with an aqueous solution of water-soluble polystyrene sulfonate (molecular weight: 70,000 g/mol; concentration: 61.4 mg/100 ml; pH = 2) by the spray method (contact time with solution: 30 seconds; distance from the spray: 10 cm; sprayed with a polymer solution and then washed with an aqueous solution adjusted to pH 2 for 20 seconds). In order to regulate the film thickness, the aqueous polymer solutions contained sodium chloride (2.92 g/100 ml). In Fig. 6, alternate laminates of polyallylamine hydrochloride and polystyrene sulfonate (72 pairs) were provided on the alternate laminates of polyacrylic acid and polyethylene glycol (9.5 pairs). Linear growth of alternately laminated films of polyallylamine hydrochloride and polystyrene sulfonate was observed.

### Example 18: Production of self-supported alternately laminated films

When the laminate obtained in Example 17 was immersed in an aqueous solution of pH about 7.0, a hydrogen bond in the under layer was attenuated (Fig. 1), the hydrogen-bonded film portion was selectively dissolved in an aqueous solution, and the alternately laminated films formed via electrostatic interactions was spontaneously released in the aqueous solution (Fig. 7). The separated film shown in Fig. 7 was dried, and the alternately laminated film was recovered. The thickness of the obtained alternately laminated film was estimated to be 200 nm or smaller based on Fig. 6, and the film was transparent and self-supported in the air (Fig. 8). The spontaneously separated film shown in Fig. 7 can be mounted onto a transmission electron microscope grid, and such membrane can be subjected to TEM analysis.

### Examples 19 and 20: Measurement of thickness of alternately laminated films formed via electrostatic interactions remaining undissolved

On the alternately laminated layer of polyacrylic acid and polyethylene glycol (9.5 pairs), alternate laminates of polyallylamine hydrochloride and polystyrene sulfonate (20 pairs (Example 19) and 80 pairs (Example 20)) were provided by the method described in Example 17. Upon immersing of part of the laminate in an aqueous solution of pH about 7.0, the portion that was not immersed in the solution remained being immobilized on the film, and the portion that was immersed therein selectively floated in the aqueous solution (Fig. 9D). In order to completely terminate the elution of the hydrogen-bonded film, the film was allowed to stand for 3 hours, and the entire laminate was then pulled out in the air. The end of the alternately laminated film formed via electrostatic interactions remaining undissolved (Fig. 9A) was observed using the atomic force microscope, and the difference between the height of the alternately laminated film and the height of the support (i.e., the thickness of the alternately laminated films formed via electrostatic interactions) was measured (Fig. 9B and 9C). As a result, the alternately laminated films of polyallylamine hydrochloride and polystyrene sulfonate (20 pairs) were found to be 55 nm and those (80 pairs) were found to be 200 nm. In the case of the ends of the folded thin films, multiple differences were observed in the thickness; i.e., the thickness of a portion comprising two thin films was twice the aforementioned thickness (i.e., 110 nm in the case of 20 pairs and 400 nm in the case of 80 pairs).

### Example 21: Case that a self-supported alternately laminated film is not obtained

As with the case in Example 17, an alternately laminated film formed via electrostatic interactions was formed on the surface of the hydrogen-bonded film. In this example, thin alternately laminated films of polyacrylic acid and polyethylene glycol were provided (5.5 pairs) (Fig. 10). In such a case, when this laminate was immersed in an aqueous solution of pH 5 to 8, a hydrogen bond in the under layer was attenuated (Fig. 1), the hydrogen-bonded film was selectively dissolved in the aqueous solution, and the thickness of the entire film was decreased (Fig. 11), although the alternately laminated film formed via electrostatic interactions was not released in the aqueous solution. This is considered to result from excessively thin hydrogen-bonded film thickness and to result from electrostatic interactions between the electrostatically interactive film and the support. This indicates that the number of lamination of hydrogen-bonded films could influence the occurrence of separation.

### Example 22: Introduction of proteins

The support was coated with the hydrogen-bonded film comprising polyacrylic acid and polyethylene glycol by the aforementioned method (pH 2.00). On the surface of the hydrogen-bonded film having polyacrylic acid (polyanion) as the outermost layer, a polymer electrolytic laminate comprising polyallylamine hydrochloride (polycation) and polysulfonate (polyanion) was provided (pH 2.00). A polymer having a opposite charge against the protein of interest was immobilized as the outermost layer. Myoglobin (polycation) was immobilized by preparing the polymer electrolytic laminate so as to comprise polysulfonate as the outermost layer and immobilizing peroxidase dissolved in an aqueous solution (1 mg/1 ml) on the surface thereof (pH 4.00). When this laminate was immersed in an aqueous solution adjusted to pH 7.00, elution of the hydrogen-bonded film and spontaneously release of a protein-containing polymer laminate were visually observed.

### Example 23: Self-supported tubular thin film

The hydrophobic inner wall surface of a tubular substance (e.g., a glass tube) was designated as a support, and the tubular inner wall surface was coated with the hydrogen-bonded film comprising polyacrylic acid and polyethylene glycol (pH 2.00). A polymer solution was brought into contact with the tubular inner wall surface by immersing the tube in the polymer solution. In order to remove excessively adsorbed polymers following the contact with the polymer solution, a step of rinsing with an aqueous solution of pH 2.00 was carried out as with the case of Examples 1 to 4. A polymer electrolytic laminate comprising polyallylamine hydrochloride and polysulfonate was provided on the hydrogen-bonded film surface comprising polyacrylic acid as the outermost layer in the same manner as in the case of the hydrogen-bonded film. The inner wall of the glass tubular substance was immersed in an aqueous solution adjusted to pH 7.00. The tube was allowed to stand therein, and release of the tubular self-supported thin film in the aqueous solution was visually observed. This indicates that, upon immersing in an aqueous solution, water molecules penetrated the polymer electrolytic laminate and reached the hydrogen-bonded film, which resulted in elution of the hydrogen-bonded film, disintegration of the hydrogen-bonded film, and automatic release of the polymer electrolytic laminate comprising polyallylamine hydrochloride and polysulfonate.

All publications, patents, and patent applications cited herein are incorporated herein by reference in their entirety.

### Industrial Applicability

The present invention can provide a self-supported thin film that can be put to a wide range of applications. In particular, a thin film comprising substances sensitive to acidic or alkaline conditions, temperature changes, or organic solvents and that are fragile can be obtained without losing biological, electronic, magnetic, or optical properties. Since such thin films can be obtained with low damage, use of inorganic substances that had to be added for the purpose of enhancing mechanical strength of thin films is not required. Accordingly, more substances can be used for providing self-supported films, such films can be put to extensive applications, such as medical engineering such as cell culture sheets or capsules for controlled-release drugs, medical or biological fields, or chemical technology such as production of self-supported separation films.

## Claims

1. A laminate comprising a support, a multilayer film assembled via a hydrogen bond thereon, and a thin film thereon.

2. The laminate according to claim 1, wherein the multilayer film assembled via a hydrogen bond is dissolved in a pH-adjusted aqueous solution and the thin film is separated.

3. The laminate according to claim 1, wherein the multilayer film formed on the support comprises two or more components.

4. The laminate according to claim 1, wherein the multilayer film formed on the support comprises a physiologically acceptable polymer.

5. The laminate according to claim 1, wherein a thickness of the multilayer film formed on the support is between 0.0005 µm and 10 µm.

6. The laminate according to claim 1, wherein the thin film comprises in its structure an alternately-laminated film comprising polyelectrolyte.

7. The laminate according to claim 1, wherein the thin film comprises at least one member selected from among proteins, cells, colloidal particles, clay minerals, semiconductor particles, and pigment molecules.

8. The laminate according to claim 1, which comprises a barrier layer between the multilayer film and the thin film.

9. A laminate further comprising a multilayered thin film on the thin film of the laminate according to claim 1.

10. A self-supported thin film obtained from the laminate according to claim 1.

11. A method for producing the laminate according to claim 1 comprising allowing a support to come into contact alternately with two or more types of solutions comprising substances to be laminated to form a multilayer film and forming a thin film via a method selected from among vapor deposition, spin coating, and layer-by-layer adsorption.

12. The production method according to claim 11, wherein the thin film is formed by allowing the support having the multilayer film to come into contact alternately with two or more types of solutions comprising substances to be laminated.

13. A method for producing a self-supported thin film comprising immersing the laminate according to claim 1 in a pH-adjusted aqueous solution to dissolve the multilayer film and to separate the thin film from the support.

14. A thin film for cell culture obtained from the laminate according to any one of claims 1 to 9.

15. A capsule for a controlled-release drug obtained from the laminate according to any one of claims 1 to 9.

16. A separation film obtained from the self-supported thin film according to claim 10.

17. A device for catalytic reactions obtained from the self-supported thin film according to claim 10.

18. A sample slice for transmission assay obtained from the self-supported thin film according to claim 10.

19. A tubular self-supported thin film obtained from the self-supported thin film according to claim 10.
